# EUROPÄISCHE PATENTSCHRIFT

(11) **EP 0 350 973 B1**
(45) Veröffentlichungstag und Bekanntmachung des Hinweises auf die Patenterteilung: **05.11.1997**
(21) Anmeldenummer: 89116295.0
(22) Anmeldetag: 24.08.1984
(51) Int. Cl.: A61K 51/00, A61K 51/12, A61K 45/05

(54) **Mittel und Erzeugnis für die Diagnose und Therapie von Tumoren sowie zur Behandlung von Schwächen der zelligen und humoralen Immunabwehr**
Means and product for the diagnosis and therapy of tumours and for the treatment of weaknesses of the cellular and humoral immune system
Moyen et produit pour le diagnostic et la thérapie de tumeurs ainsi que pour le traitement de déficiences du système immunitaire cellulaire et humoral

(30) Priorität: 26.09.1983 DE 3334751; 07.10.1983 DE 3336583; 24.01.1984 DE 3402312
(43) Veröffentlichungstag der Anmeldung: 17.01.1990
(62) Teilanmeldung aus: 84110118.1
(73) Patentinhaber: Ehrenfeld, Udo, Dr., D-93053 Regensburg (DE)
(72) Erfinder: Ehrenfeld, Udo, Dr., D-93053 Regensburg (DE)
(74) Vertreter: Kraus, Walter, Dr.

(56) Entgegenhaltungen:
- CHEMICAL ABSTRACTS, Band 98, Nr. 25, 20. Juni 1983, Seite 425, Nr. 213978k, Columbus, Ohio, US; W.E. FOGLER et al.: "The activation of tumoricidal properties in macrophages of endotoxin responder and nonresponder mice by liposome-encapsulated immunomodulators", & J. RETICULOENDOTHEL. SOC. 1983, 33(3), 165-174
- THE JOURNAL OF IMMUNOLOGY, Band 130, Nr. 4, April 1983, Seiten 1500-1502, The
- American Association of Immunologists, US; G. LOPEZ-BERESTEIN et al.: "The activation of human monocytes by liposome-encapsulated muramyl dipeptide analogues"
- BIOLOGICAL ABSTRACTS, Band 76, Nr. 7, 1983, Ref. Nr. 49355; B. CHARLEY et al.: "In vitro effects of lipopolysaccharides and mycobacterial cell wall components on swine alveolar macrophages", & RES. VET. SCI. 34(2), 212-217, 1983
- BIOLOGICAL ABSTRACTS, Band 62, Nr. 6, 1975, Ref. Nr. 33727; E. LEDERER et al.: "Cell walls of mycobacteria and related organisms: Chemistry and immunostimulant properties", & MOL. CELL. BIOCHEM. 7(2), 87-104, 1975
- CHEMICAL ABSTRACTS, Band 91, Nr. 23, 3. Dezember 1979, Seite 57, Zusammenfassung Nr. 186716v, Columbus, Ohio, US; A. WAKUI et al.: "Evaluation of the immunomodulator administration time in cancer chemoimmunotherapy", & GAN TO KAGAKU RYOHO 1979, 6(RINJI ZOKAN 2), 219-230

## Beschreibung

Die Erfindung betrifft eine Mittel sowie ein Erzeugnis, das das Mittel enthält, für die Diagnose und Therapie von malignen Tumoren sowie zur Behandlung von Schwächen der zelligen und humoralen Immunabwehr.

Die Diagnose und die Therapie maligner Tumoren ist trotz intensiver Forschung heute noch schwierig. Es ist fast unmöglich, maligne Tumoren im Frühzustand zu erkennen, und bis heute steht kein Verfahren zur Verfügung, mit dem eine Frühdiagnose durchgeführt werden kann.

Die Therapie maligner Tumoren ist - wie allseits bekannt ist - nicht zufriedenstellend.

U. Ehrenfeld berichtet (Krebsgeschehen 5, 132 ff. (1979)) über die cancerotoxische Wirkung eines Gemisches aus Acetaldehyd und Ethanol. Das Gemisch enthält 3 bis 10 g Acetaldehyd pro 1000 g Ethanol. Es zeigte sich jedoch, daß die Wirkung dieses Gemisches bei der Behandlung von soliden malignen Tumoren wie auch von Metastasen nicht ausreichend ist.

Es ist bekannt, Liposomen als Träger für Arzneimittel und Markierungen zu verwenden und diese in bestimmten Organen anzureichern. Es wurde festgestellt, daß intravenös angewendete Liposomen als Arzneimittelträger nicht kontinuierlich durch die kapillaren Wände hindurchgehen können und rasch von phagocytierenden Zellen aufgenommen werden (G. Poste, Biol. of the Cell 47, 19 (1983)).

Derartige Liposomen können den Kreislauf auch nicht verlassen, und daher sind sie ungeeignet, als direkte Träger von Markierungen oder Medikamenten für Tumoren zu dienen. Eine einfache Anwendung von Liposomen durch Inhalation scheiterte in der Vergangenheit daran, daß die Liposomen nicht in der verfügbaren Zeit die Alveolenwand der Lunge durchdringen können.

In den Literaturstellen Chemical Abstracts, Bd. 98, Nr. 25, 20. Juni 1983, S. 425, Nr. 213978k, Columbus, Ohio, US; W.E. Fogler u.a.: "The activation of tumoricidal properties in macrophages of endotoxin responder and nonresponder mice by liposome-encapsulated immunomodulators" & J. Reticuloendothel. Soc. 1983, 33(3), 165-174; und The Journal of Immunology, Bd. 130, Nr. 4, April 1983, S. 1500-1502, The American Association of Immunologists; G. Lopez-Berestein u.a.: "The activation of human monocytes by liposome-encapsulated muramyl dipeptide analogues" werden ³H-markiertes und liposomiertes MDP bzw. liposomiertes Fluorescein-LPS beschrieben. Als Immunmodulatoren werden genannt: Lipopolysaccharide, Lymphokine, Makrophagen-Aktivierungsfaktor Muramyldipeptid und verschiedene Derivate von Muramyldipeptid. In diesen Literaturstellen werden jedoch keine Arzneimittel und keine Diagnosemittel beschrieben.

Es wurden weiterhin Versuche unternommen, Liposomen zusammen mit Immunmodulatoren intravenös zu injizieren. Diese Methode schien bei Tieren erfolgreich. Da die Liposomen, wie oben erwähnt, ohne Zellvermittlung den Kreislauf nicht verlassen können, ist diese Methode aber bislang für die Diagnose und Therapie beim Menschen nicht anwendbar.

Es besteht ein großer Bedarf an einem Mittel, welches in der Lage ist, zellige Wände zu durchdringen, wie beispielsweise die Zellwände der Lunge, der Blutgefäße, der Lymphgefäße, so daß es möglich wird, daß dieses selbst oder gegebenenfalls ein Arzneimittel tragend unmittelbar an malignen Tumoren oder zur Behebung zelliger Abwehrschwächen wirkt.

Die Diagnose maligner Tumoren ist oft extrem schwierig. Röntgenologisch lassen sich maligne Tumoren erst nachweisen, wenn sie eine bestimmte Größe erreicht haben. Die Bildung von kleinen Tumoren und Metastasen kann oft nicht nachgewiesen werden, was mit sich bringt, daß eine Früherkennung von Tumoren nicht möglich ist und daß maligne Tumoren bei chirurgischen Eingriffen oft nur unvollständig entfernt werden können. Es besteht daher ein Bedarf an einem Diagnoseverfahren, mit dem auch kleine Tumoren auf einfache und leichte Weise nachgewiesen und erkannt werden können.

In den letzten Jahren werden in zunehmender Maße Immunabwehrschwächen beobachtet, die aus vielerlei Gründen auftreten. Die Herabsetzung der Abwehr von Infektionskrankheiten wird teilweise durch Umweltgifte,wie Blei, Schwefeloxide, Stickoxide, Cadmium,und durch die Kupferverarmung der Nahrung hervorgerufen.

Zur Behandlung der humoralen Immunabwehrschwäche werden Immunglobulinkonzentrate verwendet. Zur Behandlung der zelligen Immunabwehrschwächen werden HLA-gleiche Zellkonzentrate verwendet. Diese stehen jedoch nur in äußerst geringem Ausmaß zur Verfügung und besitzen eine extrem kurze Lebensdauer, weshalb die letztgenannte Therapie meist erfolglos bleiben muß.

Der vorliegenden Erfindung liegt die Aufgabe zugrunde, ein Mittel und ein Erzeugnis für die Diagnose und die Therapie maligner Tumore zur Verfügung zu stellen. Das Mittel soll hochwirksam sein und daher in geringerer Konzentration als die bekannten Mittel verabreicht werden können. Das erfindungsgemäße Mittel soll bewirken, daß der Organismus des Patienten weniger stark belastet wird, und weiterhin soll seine Verabreichung auf einfache Weise möglich sein.

Erfindungsgemäß soll ein Mittel zur Verfügung gestellt werden, mit dem man die Immunabwehrschwächen bei Menschen und Tieren erfolgreich behandeln kann. Das Mittel soll unbegrenzt verfügbar sein und einfach anzuwenden sein. Es soll gut dosierbar sein. Für das Mittel sind ungiftige Antidots im Handel erhältlich; Cortisol und Mittel, die eine Cortisolausschüttung bewirken, setzen es außer Kraft. Überraschenderweise wurde jetzt gefunden, daß Immunmodulatoren in der Lage sind, vor allem bei oraler Anwendung, insbesondere bei parenteraler Anwendung, durch Zellwände hindurchzutreten. Dadurch wird ein neuartiger Erkennungsweg und eine neuartige Behandlungsweise von malignen Tumoren möglich.

Gegenstand der Erfindung ist ein Mittel zur Diagnose und Therapie von bösartigen Tumoren sowie zur Therapie von Schwächen der zelligen und humoralen Immunabwehr, das dadurch gekennzeichnet ist, daß es neben üblichen Trägerstoffen und/oder Verdünnungsmitteln mindestens eine Verbindung aus der Gruppe
(1) einem markierten Immunmodulator, als spezifischer Träger für Substanzen durch zellige Gewebe hindurch zu gegebenenfalls vorhandenen Krebszellen zum dortigen Verbleib bei enteraler oder parenteraler Gabe,
(2) einem mit einem radioaktiven Strahler, einem Farbstoff oder einem Cytostatikum markierten Immunmodulator oder einem Gemisch derartig markierter Immunmodulatoren als spezifische Träger für Substanzen durch zellige Gewebe hindurch zu gegebenenfalls vorhandenen Krebszellen zum dortigen Verbleib bei enteraler oder parenteraler Gabe oder
(3) einem mit einem radioaktiven Strahler, einem Farbstoff oder einem Cytostatikum markierten Immunmodulator oder einem Gemisch derartig markierter Immunmodulatoren
   (a) in und/oder auf Liposomen als spezifische Träger für Substanzen durch zellige Gewebe hindurch zu gegebenenfalls vorhandenen Krebszellen zum dortigen Verbleib bei enteraler oder parenteraler Gabe oder
   (b) lipidiert als spezifische Träger für Substanzen durch zellige Gewebe hindurch zu gegebenenfalls vorhandenen Krebszellen zum dortigen Verbleib bei enteraler oder parenteraler Gabe oder
(4) einem Gemisch aus zwei oder mehreren der obigen Verbindungen als spezifische Träger für Substanzen durch zellige Gewebe hindurch zu gegebenenfalls vorhandenen Krebszellen zum dortigen Verbleib bei enteraler oder parenteraler Gabe
enthält.

Gegenstand der Erfindung ist weiterhin ein Erzeugnis, enthaltend
a) ein Hilfsmittel, welches neben üblichen pharmazeutisch annehmbaren Trägerstoffen und/oder Verdünnungsmitteln einen Aldehyd der Formel I

   RCHO (I)

   worin R ein Wasserstoffatom oder eine geradkettige oder verzweigte Kohlenwasserstoffgruppe mit 1 bis 4 Kohlenstoffatomen bedeutet, wobei der freie Aldehyd auch direkt oder indirekt von Stoffen metabolisch freigesetzt werden kann, und gegebenenfalls einen Alkohol der Formel II:

   RCH₂OH (II)

   worin R die oben angegebene Bedeutung besitzt,
   und
b) mindestens eine Verbindung aus der Gruppe
   (1) einem markierten Immunmodulator, als spezifischer Träger für Substanzen durch zellige Gewebe hindurch zu gegebenenfalls vorhandenen Krebszellen zum dortigen Verbleib bei enteraler oder parenteraler Gabe,
   (2) einem mit einem radioaktiven Strahler, einem Farbstoff oder einem Cytostatikum markierten Immunmodulator oder einem Gemisch derartig markierter Immunmodulatoren als spezifische Träger für Substanzen durch zellige Gewebe hindurch zu gegebenenfalls vorhandenen Krebszellen zum dortigen Verbleib bei enteraler oder parenteraler Gabe oder
   (3) einem mit einem radioaktiven Strahler, einem Farbstoff oder einem Cytostatikum markierten Immunmodulator oder einem Gemisch derartig markierter Immunmodulatoren
      (a) in und/oder auf Liposomen als spezifische Träger für Substanzen durch zellige Gewebe hindurch zu gegebenenfalls vorhandenen Krebszellen zum dortigen Verbleib bei enteraler oder parenteraler Gabe oder
      (b) lipidiert als spezifische Träger für Substanzen durch zellige Gewebe hindurch zu gegebenenfalls vorhandenen Krebszellen zum dortigen Verbleib bei enteraler oder parenteraler Gabe oder
   (4) einem Gemisch aus zwei oder mehreren der obigen Verbindungen als spezifische Träger für Substanzen durch zellige Gewebe hindurch zu gegebenenfalls vorhandenen Krebszellen zum dortigen Verbleib bei enteraler oder parenteraler Gabe
enthält, zur gleichzeitigen, getrennten oder zeitlich abgestuften Anwendung bei der Diagnose und Therapie von malignen Tumoren und zur Therapie von Immunabwehrschwächen.

Das Mittel dringt überraschenderweise leicht durch die Zellwände hindurch, insbesondere durch die Wände von Lungenalveolen, Lymph- und Blutkapillaren. Dadurch werden eine neue Diagnose und eine neuartige Behandlungsweise von malignen Tumoren ermöglicht, da die mit einem radioaktiven Strahler versehenen Immunmodulatoren unmittelbar zum malignen Tumor gehen und dort zur Erkennung und Behandlung des Tumors die höchstmögliche Wirkung erbringen.

Das erfindungsgemäße Mittel kann zur Diagnose von malignen Tumoren in vivo und in vitro verwendet werden. Die malignen Tumoren können außerhalb des Körpers diagnostiziert werden. Beispielsweise kann der Chirurg bei einer Operation malignes Gewebe entfernen und dann das lebende Gewebe außerhalb des Organismus inkubieren, so daß im flachen Anschnitt des Gewebes, z.B. bei Farbmarkierung mit Fluoresceinisothiocyanat im UV-Licht malignes Gewebes aufleuchtet und von dunklem gesundem Gewebe sofort unterschieden werden kann.

Mit dem erfindungsgemäßen Mittel lassen sich Tumorgrößenordnungen von 100 bis 10000 Zellen exakt kennzeichnen; bei zusätzlicher Sauerstoffgabe ist das Mittel in der Lage, anhand der für die Diagnostik eingebrachten minimalen radioaktiven Strahlung eine floride Entzündung infolge Strahlenzerstörung von Tumorgewebe zu erzeugen.

Als Immunmodulatoren können bei der vorliegenden Erfindung all die Verbindungen verwendet werden, die im Handel erhältlich sind und die in der Literatur beschrieben werden.

Unter "Immunmodulatoren", die auch als "Immunstimulantien" bezeichnet werden, versteht man Verbindungen oder Gemische, die das Immunsystem auf irgendeine Weise stimulieren.

Erfindungsgemäß werden bevorzugt als Immunmodulatoren solche Verbindungen verwendet, die bereits in der Therapie oder Diagnose beim Menschen eingesetzt werden. Besonders bevorzugt werden erfindungsgemäß Muramylsäuredipeptidderivate (MDP) oder Peptidoglykane oder peptidoglykanfreie Extrakte, beispielsweise von Polysaccharidstruktur, wie Extrakte aus Norcardia rubra oder Norcardia opaca,oder aus anderen Bakterien, vorzugsweise aus Bacille Calmette Guérin (BCG), sowie entgiftete Präparationen aus Salmonellen sowie lipidierte Polysaccharide, vorzugsweise lipidiert, solche aus Salmonellapräparationen, die durch Entfernung von anhängenden Gruppen entgiftet und somit für die Anwendung am Menschen für die genannten Wirkungen geeignet vorliegen,und deren Derivate, verwendet.

Die Immunmodulatoren können natürlichen Ursprungs, d.h. naturbelassen, halbsynthetisch oder synthetisch hergestellte Stoffe sein.

Die als Immunmodulatoren bezeichneten Peptidoglykane bestehen aus einem Polysaccharidskelett, welches aus Disaccharidbausteinen von N-Acetylglucosaminyl-N-acylmuramyl aufgebaut ist, in denen die Acylgruppe durch eine Acetyl-oder Glykolylgruppe gebildet wird. Diese Einheiten müßten für den gewünschten Effekt in einer ausreichenden Anzahl vorhanden sein. Außer diesen Einheiten können auch 0 bis 10% Neutralzucker vorhanden sein. Diese Peptidoglykane enthalten Peptidsubstituenten, die so weit ihrer interpeptidischen Bindungen verlustig gegangen sind, daß sie wasserlöslich geworden sind und nur noch gering fettlöslich sind.

Am Ende der Glykanketten sollten keine Peptidsubstituenten mehr vorhanden sein. Die Peptidoglykane sollen von natürlichen Lipiden, mit denen sie zusammen in Bakterienwänden vorkommen, völlig befreit sein. Diese Lipide können aber in einer bevorzugten Ausführungsform des erfindungsgemäßen Mittels durch andere Lipide, z.B. Phosphatidylcholin allein oder mit Phosphatidylserin und Cholesterol zusammen im molaren Verhältnis 8:2:10 ersetzt sein, wobei die Wasserlöslichkeit des Peptidoglykans erhalten bleiben soll.

Diese letztgenannten Lipide können als Lösungsmittel oder gebunden an Peptidoglykane vorkommen. Die N-Acetylglucosamingruppen der Peptidoglykane können teilweise desacetyliert sein. Das gesamte Molekül kann aber auch zusätzlich mit Aldehyden der Formel I

RCHO (I)

worin R ein Wasserstoffatom oder eine geradkettige oder verzweigte Kohlenwasserstoffgruppe mit 1 bis 4 Kohlenstoffatomen bedeutet, umgesetzt sein. Vorzugsweise werden sie mit Acetaldehyd umgesetzt.

Die über L-Alanin gebundenen Peptideinheiten der Peptidoglykane enthalten Sequenzen von L-Alanin → D-isoGlutamin → meso-α-ε- diamino-Pimelinsäure (DAP), wobei die DAP amidiert sein kann. An ihrer Stelle kann auch Uridindiphosphat vorhanden sein oder natürlicherweise eine andere Aminosäure, wie zum Beispiel Lysin, die ihrerseits substituiert sein kann.

Die oben genannte Änderung der Peptidoglykane, wie eine Lipidierung und Einführung anderer Peptidsubstituenten, wie zum Beispiel UDP und anderer mehr, sowie die Verwendung anderer Polysaccharidketten mit gleicher Wirkung und ähnlicher Substituenteneinführung, führen zu Lipopolysacchariden, die ebenfalls erfindungsgemäß verwendet werden können.

Die Immunmodulatoren können auch mit Humanalbumin oder/und Immunglobulin versetzt werden und so intravenös oder intralymphatisch verabreicht werden. Bevorzugt ist hierbei die Verabreichung in Liposomen.

Erfindungsgemäß können die Immunmodulatoren mit einem radioaktiven Strahler markiert sein. Die Markierung mit einem radioaktiven Strahler ist in der Literatur beschrieben (M.P. Osborne, V.J. Richardson, K. Jeyasingh und B.E. Ryman, Int. J. Nucl. Med. Biol. 6, 75 (1979)).

Beispiele für radioaktive Strahler sind die Strahler, die man üblicherweise auf dem Gebiet der Therapie und Diagnose verwendet, wie beispielsweise ^{99m}Tc, 0,97 bis 7,4 GBq (25 bis 200 mCi), da bei den anwendungsbedingt geschlossenen Inhalationssystemen über 50% der Radioaktivität im System verbleiben und hohe Lungenbelastungen unnötig sind.

Die Immunmodulatoren können weiterhin ein Cytostatikum tragen. Beispiele für Cytostatika und Metastasehemmer, die erfindungsgemäß eingesetzt werden können, sind alle derzeit als Cytostatika und Metastasehemmer bekannten Verbindungen. Beispiele hierfür sind Melphalan, Carmustin, Lomustin, Cyclophosphamid, Estramustinphosphat, Ifosfamid, Chlorambucil, Methotrexat, Tegafur, Fluorouracil sowie Antibiotika, die für diese Zwecke eingesetzt werden.

Die Immunmodulatoren können weiterhin mit einem Farbstoff markiert sein. Als Farbstoffe eignen sich beispielsweise marktübliche, im Menschen ungiftige, Aminogruppen markierende Farbstoffe, wie unter anderem Fluoresceinisothiocyanat. Von dem entsprechenden Farbstoff, z.B. Fluoresceinisothiocyanat, wird eine Anzahl von Molekülen mit der für die Therapie angegebenen Präparation des Immunmodulators gemischt, die deutlich unter der Zahl der in diesem vorhandenen Aminogruppen liegt. Dann läßt man das Gemisch bei 37°C 45 Minuten stehen.

Erfindungsgemäß ist es auch möglich, Gemische aus einem oder mehreren Immunmodulatoren, solchen, die mit einem radioaktiven Strahler, einem Farbstoff oder einem Cytostatikum markiert sind, zu verwenden.

Gemäß der vorliegenden Erfindung ist es bevorzugt, daß die markierten Immunmodulatoren nicht allein, sondern zusammen mit Liposomen oder in lipidierter Form verwendet werden.

Liposomen sind kugelförmige Gebilde aus einer oder mehreren Lipiddoppelschichten mit einem Innenraum. Derartige Bläschen lassen sich durch mechanische Feinstverteilung von Phospholipiden, wie z.B. Lecithin, in wäßrigen Medien herstellen.

Es werden Liposomen verwendet, die einzelne unilamellare Bläschen (SUV) sind und vorzugsweise aus Phosphatidylcholin : Phosphatidylserin : Cholesterol im molaren Verhältnis 8 : 2 : 10 bestehen und durch Sonication hergestellt werden. Die Lipide, aus denen sie hergestellt werden, sind im Handel erhältlich, beispielsweise von Sigma Chemical Co. Sie werden durch Säulenchromatographie gereinigt, in Ether gelöst, unter N₂ getrocknet, mit dem Immunmodulator bzw. dem beschickten Immunmodulator vermischt, in phosphatgepufferter Kochsalzlösung (PBS), beispielsweise bei pH 7,4, wieder suspendiert und dann beispielsweise 25 Minuten bei +2°C mit einem pulsierenden Branson 15 Sonicator beschallt (sonicated). Die Sonication wird im allgemeinen unter N₂ durchgeführt.

Nach der Sonication werden die Liposomen auf einer Sepharose®-4-B-Säule chromatographiert und vorzugsweise die Fraktionen der Population mit Radii unter 30 nm (300 Å) benutzt (C. Huang, Biochemistry 15, 2362 (1969)). Diese Liposomen werden dann zur Diagnostik in an sich bekannter Weise mit vorzugsweise 99mTc am Immunmodulator markiert entsprechend Osborne et al. (M.P. Osborne, V.J. Richardson, K. Jeyasingh und B.E. Ryman, Int. J. Nucl. Med. Biol. 6, 75 (1979)).

Um die radioaktive Markierung zu prüfen, wird ein Aliquot der Liposomen auf eine Sepharose-4-B-Säule gegeben und chromatographiert. Man stellt fest, daß in der Präparation 99,2% der Radioaktivität an Immunmodulator gebunden und 0,8% als freies Pertechnetat vorliegt.

Die Liposomen können mit Immunmodulatoren beschickt sein, die mit einem radioaktiven Tracer, mit einem Farbstoff, einem Cytostatikum oder mit Gemischen dieser Verbindungen beschickt sind. Derartige Liposomen sind insbesondere für die Diagnose geeignet.

Gemäß einer weiteren bevorzugten Ausführungsform werden der Immunmodulator oder die Liposomen gegebenenfalls, wie oben erläutert, beschickt in einem Lipid dispergiert. Die Dispersion erfolgt, indem man die Substanzen zusammenbringt und ebenfalls einer Beschallung unterwirft.

Abweichend von der o.g. Herstellung der Liposomen ist, daß beispielsweise:
(1) die Trocknung der hierbei in Ethanol und in Ether gelöst vorliegenden Lipide unter N₂ unterbleibt,
(2) nach der Sonication die Chromatograhpierung und die Abtrennung der Fraktionen mit Radii über 30 nm (300Å) unterbleibt, und dieses Produkt nur zur Inhalation verwendet wird.

Als Lipide kann man Phosphatidylcholin allein oder mit Phosphatidylserin und Cholesterol zusammen,beispielsweise im molaren Verhältnis 8 : 2 : 10,verwenden.

Wie oben erwähnt, ist bekannt, daß ein Gemisch aus Acetaldehyd und Ethanol eine cancerotoxische Wirkung hat. Überraschenderweise wurde jetzt gefunden, daß das erfindungsgemäße Mittel eine besonders gute Wirkung entfaltet, wenn es zusammen mit einem Hilfsmittel verabreicht wird. Das Hilfsmittel enthält neben üblichen Trägerstoffen und/oder Verdünnungsmitteln einen Aldehyd der Formel I

RCHO (I)

worin R ein Wasserstoffatom oder eine geradkettige oder verzweigte Kohlenwasserstoffgruppe mit 1 bis 4 Kohlenstoffatomen bedeutet. Es ist besonders bevorzugt, daß das Hilfsmittel zusätzlich zu dem Aldehyd der Formel I einen Alkohol der Formel II

R¹CH₂OH (II)

enthält, worin R¹ die für R gegebene Bedeutung besitzt. Die Erfindung betrifft somit auch ein Erzeugnis, welches das oben beschriebene Mittel und das oben beschriebene Hilfsmittel enthält. Überraschenderweise zeigte sich, daß bei gleichzeitiger und zeitlich abgestufter Verwendung des Hilfsmittels zusammen mit dem erfindungsgemäßen Mittel der Wirkungsgrad für das erfindungsgemäße Mittel noch wesentlich verbessert wird.

Das Hilfsmittel in dem erfindungsgemäßen Erzeugnis kann den Aldehyd als solchen in üblichen pharmakologisch verträglichen Trägern und/oder Verdünnungsmitteln enthalten. Besonders bevorzugt ist es, den Aldehyd in wäßriger und/oder alkoholischer Lösung einzusetzen. Erfindungsgemäß ist es dabei besonders bevorzugt, den jeweiligen Aldehyd zusammen mit seinem zugehörigen Alkohol zu verwenden.

Bevorzugte Hilfsmittel setzen indirekt oder direkt frei und/oder enthalten Formaldehyd/Methanol, Acetaldehyd/Ethanol, n-Propionaldehyd/n-Propanol, n-Butyraldehyd/n-Butanol, iso-Butyraldehyd/iso-Butanol, n-Valeraldehyd/n-Pentanol oder Gemische dieser Verbindungen.

Eine optimale Wirkung des neuen pharmazeutischen Präparats, u.a. eine Verbesserung der Permeabilität für beschickte Liposomen, wird offenbar dann erzielt, wenn die Konzentration des Aldehyds im Körper über längere Zeiträume hoch gehalten, vorzugsweise gleichmäßig hoch gehalten, werden kann. Es ist bekannt, daß Ethanol im menschlichen Körper zu Acetaldehyd abgebaut wird, wobei die Abbaurate des Ethanols oberhalb einer bestimmten Konzentration ebenso wie die des Acetaldehyds praktisch unabhängig von der Konzentration ist und die Abbaurate des Acetaldehyds offenbar in der gleichen Größe oder etwas geringer ist als die des Ethanols. Diese beim natürlichen Alkoholabbau auftretende Konzentration an Acetaldehyd ist aber ersichtlich nicht hoch genug.

Mit dem Hilfsmittel ist es einerseits möglich, eine hinreichend hohe Konzentration von zum Beispiel Acetaldehyd - im Körper des an Tumoren und/oder Metastasenbildung Erkrankten auszubilden, wobei mittels der bevorzugten Ausführungsform der Erfindung durch die gleichzeitige Gabe einer möglichst unschädlichen Substanz - insbesondere zugehöriger Alkohol - durch deren Abbau laufend der entsprechende Aldehyd nachgebildet wird, der die Durchtrittsverbesserung durch Zellen und Gewebe sowie die Wirkungsrichtung des lipidierten Immunmodulators zur zelligen Immunabwehr begünstigt.

Das Stoffpaar Acetaldehyd/Ethanol ist praktisch ungiftig; man kann es in geeignet hohen Dosen verabreichen. Daraus resultiert die Möglichkeit einer Dauerbehandlung, auch in Kombination mit einer Strahlenbehandlung. Das immunbiologische System wird positiv beeinflußt, und eine Kombination mit anderen Medikamenten sowie mit chirurgischen und radiologischen Maßnahmen ist möglich.

Außer dieser Mischung Ethanol/Acetaldehyd sind grundsätzlich auch andere analoge Mischungen der oben genannten Art möglich, wie Methanol/Formaldehyd, Propanol/Propanal, Butanol/Butanal. Methanol wird im menschlichen Körper wesentlich langsamer abgebaut als Ethanol, Propanol um den Faktor 2 schneller als Ethanol. Das Mittel kann jeweils nur einen bestimmten ausgewählten Aldehyd wie auch Aldehydmischungen enthalten. Die Anwendung der Aldehyde ist nicht zwingend mit der Anwesenheit der entsprechenden Alkohole gekoppelt. Auch wäßrige Lösungen der Aldehyde können eingesetzt werden. Anstelle der freien Aldehyde können erfindungsgemäß auch solche Aldehydderivate zum Einsatz kommen, die im Stoffwechsel des mit dem erfindungsgemäßen pharmazeutischen Mittel Behandelten den freien Aldehyd bilden. Geeignete Aldehydderivate sind beispielsweise die Acetale oder Halbacetale oder Kondensationsprodukte, die ebenfalls als solche oder in gelöster Form (Wasser oder Alkohole) wie auch in Mischungen mit den Aldehyden und/oder Alkoholen Verwendung finden können.

In einer bevorzugten weiteren erfindungsgemäßen Ausführungsform enthält das Hilfsmittel geringe Mengen (weniger als 0,05 Gew.-%) an Peroxiden, wobei insbesondere die hier stofflich verwandten Peroxide in Betracht kommen, insbesondere H₂O₂ und/oder das Aldehydperoxid bzw. Hydroxyhydroperoxid sowie das Peroxid der zugehörigen Carbonsäure. Durch den Gehalt an Peroxiden wird die antitumorale Wirkung noch weiter verbessert.

Die Konzentration des Aldehyds im erfindungsgemäßen Präparat ist einerseits durch dessen Verträglichkeit und andererseits durch die zu verabreichende Dosis bestimmt. Für das Paar Ethanol/Acetaldehyd ist eine Acetaldehydkonzentration im Alkohol unter 2 x 10⁻⁴ Mol/Liter häufig in der Wirkungsweise unbefriedigend langsam. Die Wirkung steigt mit steigender Aldehydkonzentration und ist nach oben hin in der Regel durch möglicherweise eintretende Unverträglichkeit des Acetaldehyds im Einzelfall begrenzt. In der Praxis bewährt haben sich beispielsweise Ethanol/Acetaldehydlösungen mit 5 x 10⁻² Mol bis 1 Mol Acetaldehyd pro Liter Ethanol, wobei diese Mischungen in einer Dosis von beispielsweise 10 bis 150 cm³ pro Tag Verwendung finden können.

Es ist bevorzugt, daß das Hilfsmittel 10 bis 40 g Aldehyd pro 1000 g Alkohol, besonders bevorzugt 15 bis 30 g Aldehyd pro 1000 g Alkohol, enthält. Im allgemeinen wird das Hilfsmittel für die Verabreichung mit Wasser verdünnt. Die alkoholische Lösung kann mit Wasser beliebig verdünnt werden. Beispielsweise kann man ein Volumen der alkoholischen Lösung mit 1 bis 10 Volumen, vorzugsweise 2 bis 5 Volumen, Wasser verdünnen.

Das Hilfsmittel wird bevorzugt oral in Form der wäßrigen Lösung verabreicht und vom Patienten getrunken. Das Hilfsmittel kann auch parenteral, zum Beispiel durch Infusion, verabreicht werden. Die Zubereitung von Infusionslösungen ist dem Fachmann geläufig und kann auf einfache Weise erfolgen.

In dem erfindungsgemäßen Erzeugnis bzw. Kit können die beiden Bestandteile jeweils auf unterschiedliche Weise kombiniert sein. Das Hilfsmittel kann in einer für die orale Verabreichung und/oder für die parenterale Verabreichung geeigneten Form vorliegen. Beispielsweise kann das Mittel in Form von Trinkampullen vorliegen, oder es kann in Form von Trinkampullen, die mit Wasser verdünnt werden, vorliegen. Das erfindungsgemäße Mittel, zum Beispiel Immunmodulator-Liposomen, kann in einer für die orale und/oder parenterale Verabreichung geeigneten Form vorliegen. Die Kombination wird entsprechend dem Zweck des Erzeugnisses zubereitet werden. Soll das Erzeugnis für die Therapie verwendet werden, wird das Erzeugnis die gleiche Zahl von Dosiseinheiten an Hilfsmittel und Mittel, zum Beispiel Liposomen, enthalten. Die Zahl kann jedoch auch variieren. Es ist beispielsweise möglich, daß ein Erzeugnis, welches für die Diagnose gedacht ist, mehrere Dosiseinheiten des Hilfsmittels und nur eine oder zwei Dosiseinheiten der Liposomen enthält. Die richtige Kombinationsauswahl des Verhältnisses der Dosiseinheiten an Hilfsmittel und Liposomen ist dem Fachmann geläufig und richtet sich nach der beabsichtigten Verwendung.

Das Hilfsmittel kann auf einfache Weise durch einfaches Mischen der Bestandteile hergestellt werden. Der ausgewählte Aldehyd wird mit pharmakologisch verträglichen Trägern und/oder Verdünnungsmitteln, gegebenenfalls zusammen mit dem Alkohol, vermischt.

Es ist jedoch bevorzugt, das Hilfsmittel herzustellen, indem man einen Alkohol der Formel II

RCH₂OH (II)

worin R ein Wasserstoffatom oder eine geradkettige oder verzweigte Kohlenwasserstoffgruppe mit 1 bis 4 Kohlenstoffatomen bedeutet, mit energiereicher Bestrahlung unter Zutritt von Sauerstoff zu bestrahlen.

Als energiereiche Bestrahlung kann beispielsweise γ -, UV-, Röntgen- oder Elektronenbestrahlung eingesetzt werden. Es können dabei die ausgewählten Alkohole als solche, aber auch Alkohol/Wasser-Mischungen Verwendung finden, wobei als Ausgangsmaterial hochkonzentrierte Alkohol/Wasser-Mischungen besonders bevorzugt sein können. Die Bestrahlung erfolgt unter Zutritt von Sauerstoff, bevorzugt unter Luftzutritt.

Ein für die Praxis besonders wichtiges und wirksames Antitumormittel läßt sich beispielsweise dadurch herstellen, daß man 96%iges Ethanol in Gegenwart von Sauerstoff energiereicher Bestrahlung der genannten Art aussetzt, bis sich die gewünschte Menge an Acetaldehyd gebildet hat. Die Lösung enthält dann im wesentlichen neben viel Ethanol den Acetaldehyd zusammen mit Peroxiden, wie H₂O₂ oder Acetoperoxid, oder Spuren von Peressigsäure sowie Essigsäure. Die zuletzt genannten Substanzen verbessern die Wirkung der immunmodulatorbeschickten Liposomen wesentlich. Überraschenderweise zeigte sich, daß eine für Ethanol/Acetaldehyd typische Vermehrung der Makrophagen, T-Zellen, T-Helferzellen und Killerzellen gefunden wurde und dieser Effekt die Wirkungsrichtung der Immunmodulatoren von einer vorwiegenden B-Zellstimulation, wie sie bekannt ist, auf eine Vermehrung der Makrophagen, T-, T-Helfer- und natural-Killer-Zellen umlenkt, so daß bis dahin unbekannte große Zahlen dieser Zellen im Menschen erzeugt werden. Dies wird in der vorgenannten Literaturstelle von U. Ehrenfeld nicht beschrieben, es lag auch nicht nahe.

Zur immunologischen medikamentösen Behandlung werden vorzugsweise Immunmodulatoren lipidiert, vorzugsweise in Liposomen, steril in physiologischer Kochsalzlösung suspendiert, per Inhalation, nach vorheriger oraler Gabe eines Cocktails aus vorzugsweise Ethanol/Acetaldehyd in Wasser, verabreicht. Dadurch kommt es zu einer eindeutig feststellbaren Verminderung der Krebsmassen ohne nachteilige Nebenwirkungen.

Falls der Immunmodulator wie o.g. mit einem radioaktiven Strahler markiert wird, kann die Sichtbarmachung der Organ-Verteilung von 99mTechnetium-markierten lipidierten Immunmodulatormolekülen vorzugsweise unter Verwendung einer externen γ-Kamera im Bild festgehalten werden.

Gemäß einer besonders bevorzugten erfindungsgemäßen Ausführungsform wird zusammen mit dem Cocktail und den strahlermarkierten Immunmodulator-Liposomen "SmIL" ein Mittel verabreicht, welches die Sauerstoffabgabe im Bereich der malignen Tumoren steigert. Dadurch wird gleichzeitig die therapeutische Bestrahlungswirkung heraufgesetzt. Als solche Mittel, die die Sauerstoffabgabe im Bereich der malignen Tumoren steigern, kann man alle derartigen Mittel verwenden, für die diese Wirkung bereits bekannt ist. Beispiele hierfür sind Inositolhexaphosphat, Glycerindiphosphat und andere Substanzen, von denen bekannt ist, daß sie in das Häm eingebaut werden können und dort diesen Effekt besitzen. Die Bestrahlungsbehandlung wird auf den Bestimmungsort beschränkt, die Bestrahlung individuell dosierbar.

Zwischenphasen bzw. Unterbrechungen der Bestrahlungsbehandlung werden durch die Behandlung mit lipidierten Immunmodulatoren genutzt.

Es war überraschend und hat nicht nahegelegen, daß sich die SmIL in einem solchen Ausmaß am und im malignen Tumor ansammeln. Hierdurch wird eine Behandlung zahlreicher verschiedener Tumoren möglich, und es wird auch möglich, allerkleinste Tumoren im Körper von Säugetieren, insbesondere von Menschen, nachzuweisen und zu diagnostizieren.

Gemäß der vorliegenden Erfindung ist es somit erstmals möglich, maligne Tumoren auf einfache Weise, ohne daß der Patient belastet wird, bei Säugetieren, insbesondere bei Menschen, zu diagnostizieren und auch zu heilen. Erfindungsgemäß ist es möglich, die Tumoren gezielt zu bestrahlen, fast ohne daß Nachbargewebe schädigt wird, und weiterhin kann man Medikamente, zum Beispiel die Cytostatica oder Immunmodulatoren, erfindungsgemäß an den Ort bringen, wo sie tatsächlich wirken sollen.

Für die Behandlung ist eine Ernährung mit weitgehend glucose- und stärkefreier Kost erforderlich. Lokal wird der Effekt der Wirkkombination aufgehoben durch Glucose, Zucker, Stärke, Vitamin C in hohen Dosen und Vitamin B₁, allgemein durch Cortison und Antihistaminika.

An jedem Behandlungstag wird eine halbe bis eine volle Dosis des Aerosols inhaliert und eine Dosis des Cocktails getrunken. Die Angaben für das Aerosol und für den Cocktail bezeichnen eine Tagesdosis.

Das Aerosol: Die Liposomen sind einzelne unilamellare Bläschen, die aus Phosphatidylcholin : Phosphatidylserin : Cholesterol im molaren Verhältnis 8 : 2 : 10 bestehen und Radii unter 30 nm (300 Å) haben, 5 mg davon sind suspendiert in 5 ml physiologischer Kochsalzlösung zur Inhalation. Die Liposomen tragen einen Immunmodulator,zum Beispiel aus Nocardia opaca 6 µg/ml, der sich außen auf den Liposomen befindet. Zur scintigraphischen Diagnostik und zur Strahlentherapie trägt der Immunmodulator eine Markierung von 0,93 bis 1,85 GBq (25 bis 50 mCi) von 99m Technetium- Pertechnetat, zur Farbdiagnostik von Krebs ausschließlich oder zusammen mit dem Strahler einen geeigneten Farbstoff. Zur Immunbehandlung von malignen Tumorerkrankungen und zur Therapie von Zuständen zelliger und humoraler Immunabwehrschwäche trägt der Immunmodulator keinen Strahler.

Der Cocktail: 25 bis 50 ml einer Kombination aus Ethanol 96% : Acetaldehyd puriss. im Verhältnis 1000 ml : 40 ml werden mit 250 bis 500 ml Leitungswasser verdünnt.

Es sind kleinste Tumormengen exakt darstellbar. Die Tumorgröße eines Malignoms ist für den Erfolg der Darstellung ohne Bedeutung. Phosphatidylcholin (Lecithin) kommt in der Lunge physiologischerweise mit Fettsäuren abgesättigt vor. Es dient zur Stabilisierung der Alveolenwände. Als Lipid an den Immunmodulatormolekülen dient es vor allem zur Passageerleichterung für diese; Ethanol und Acetaldehyd sind als Porenweitsteller an Membranen im lebenden Gewebe bekannt und unterstützen diesen Effekt des Lecithins.

Nach der Passage der Alveolenwand der Lunge wird der lipidierte Immunmodulator über Blut- und Lymphgefäße zu den malignen Tumoren gebracht, wo er hauptsächlich durch einen aktiven Vorgang der lebenden Krebszellenwand in ihr gebunden wird. Der Immunmodulator ist dort mit üblichen Färbemethoden histologisch in der Art von Granula nachweisbar, gängige Lipidfärbungen zeigen nur minimale sporadische Anzeichen für Lipid. Die Lipide gehen wohl bei den Passagen der zelligen Wände weitestgehend verloren.

Fluoresceinisothiocyanat oder 99mTc bleiben bis zur Krebszellenwand an den Immunmodulator gebunden und sind dort fluoreszenzmikroskopisch bzw. autoradiographisch nachweisbar.

Dargestellt wurden mit dem Test anhand der externen γ-Kamera verschiedene Melanosarkome, Plattenepithelkarzinome und Adenokarzinome der weiblichen Brust. Die Tests wurden außerhalb der Universitätsklinik in einem Krankenhaus mit einfachen Methoden wiederholt und waren für die dort geprüften Immunmodulatoren aus BCG und Nocardia problemlos mit denselben Resultaten reproduzierbar.

Knochenumschlossene Metastasen waren mit dem Test nicht nachweisbar, bei teilweisem Herausragen aus dem Knochen war die Abbildung möglich. Hirnmetastasen sind abzubilden, jedoch muß hier auf die Cocktailgabe verzichtet werden, da dieser sofort umfangreiche Kolliquationsnekrosen bei Hirnmetastasen induziert. Tumoren und Metastasen außerhalb der Basalmembran der Haut sind nicht abzubilden.

Bei intratumoraler Verabreichung von SmIL erfolgte im Gesunden nur Diffusionsabbildung, bei malignem Gewebe kam es zur selben langfristigen Bindung und Abbildbarkeit über Tage, wie nach Inhalation des SmIL.

Der lokale Nekrotisierungsvorgang von Tumorgewebe nach Inhalation von SmIL mit lokaler Entzündung und resorptiver Temperatursteigerung auf 38,5°C war mit Nocardia und BCG bei Gabe des Cocktails und mehrstündiger Sauerstoffinsufflation unter Feinnadelbiopsiekontrolle stets reproduzierbar.

Anläßlich der SmIL-Gabe zu den Tests mit der γ-Kamera kam es wie bei der o.g. Immunbehandlung stets zu Leukozytosen mit Steigerung der Zahlen der Makrophagen, Rieder-, T-, T-Helfer- und natural-Killer-Zellen sowie der lymphatischen Plasmazellen. Bei einer jungen Frau führte die Inhalation von SmIL (nach Cocktailgabe, wie in den vorgenannten Fällen) zu einer Steigerung der Leukozyten im Blut von 6000 auf 25000/µl ohne Temperaturanstieg oder andere Krankheitszeichen.

Bei Schwächen der zelligen und humoralen Immunabwehr stiegen vor allem die Zahlen und die Aktivität der Makrophagen, T- und T-Helfer-Zellen auf das Doppelte der Ausgangswerte nach Gabe des Cocktails und auf das 6fache nach zusätzlicher Inhalation des lipidierten Immunmodulators bei Verschiebung des Quotienten T-Helfer-/T-Suppressor-Zellen zugunsten der T-Helfer-Zellen; B-Zellen und Immunglobulinproduktion stiegen ebenfalls an. Diese Effekte treten am Gesunden und am Krebskranken gleichermaßen auf. Die in der Norm bei 140/µl liegende Zahl der Riederzellen und Monozyten stieg nach Gabe des Cocktails aus Ethanol/Acetaldehyd/Wasser auf das Doppelte; wenn zum Cocktail Muramyldipeptid in Liposomen inhaliert wurde (30 µg in 5 mg), auf 900/µl und, wenn zum Cocktail Immunmodulator aus Nocardia (ImmL) in Liposomen (30 µg in 5 mg) inhaliert wurde, auf Werte um und über 2000/µl. Bei alten und mit Leberzirrhose kranken Menschen lagen die Werte etwa auf der Hälfte der Werte von jungen Menschen.

Die Immunantwort in den so behandelten Fällen ist hinsichtlich der zelligen und humoralen gesteigerten Reaktion konstant.

Eine Erschöpfung des Immunsystems kam nicht zur Beobachtung, ebensowenig Überreaktionen und Entartungszeichen. Die Verträglichkeit erscheint ausgezeichnet.

Die nach Einsatz des beanspruchten Mittels erfolgende Rückbildung von Lebermetastasen binnen Tagen bis Wochen führte stets rasch zu einer deutlichen Besserung des Allgemeinzustandes der Patienten.

Nach Gabe von Acetaldehyd kommt es an den Zelloberflächen bekanntermaßen zur Ankopplung an NH₂-Gruppen, gelegentlich zu Senkungen des Blutzuckerspiegels, stets setzte die β-Oxidation von Fettsäuren ein. Die bei der Lipolyse anfallenden Stoffwechselprodukte wirken zum Teil ähnlich wie Acetaldehyd.

Die Phagocytoseleistung der Makrophagen beim Vernichten von Zellen maligner Tumoren steigert sich bei Anwendung von Acetaldehyd und ImmL in der genannten Weise signifikant. Das Verschwinden von Hautmetastasen, Lebermetastasen und der Umbau von Lungenmetastasen in lufthaltige Strukturen kam zur Beobachtung. An den Oberflächen maligner Tumoren zeigte sich eine signifikante Makrophageneiterung in der Histologie. Der Effekt von Röntgenbestrahlung auf Tumorgewebe wird durch Acetaldehyd/Ethanol verbessert. Die Wirkung von Acetaldehyd und ImmL in der o.g. Anwendungsart ist mengenabhängig, rascher wachsende bösartige Tumoren sprechen besser an als langsam wachsende. Die Wirkung der erfindungsgemäß bevorzugten Kombination Ethanol/Acetaldehyd in wäßriger Lösung zusammen mit Liposomen, die markiert und/oder mit Medikamenten, vorzugsweise Immunmodulatoren, beschickt sind, wird im folgenden beispielhaft an der Beschreibung des pathophysiologischen Bildes von Krebspatienten erläutert. Die Abweichungen beim Einsatz der erfindungsgemäßen Kombination zur Erkennung und Behandlung zum Unterschied der unbehandelten Krebspatienten lassen sich wie folgt zusammenfassen:

### Erkennung:

Die Markierung betrifft malignes Tumorgewebe.

### Behandlung:

a) subjektiv (Patientenurteil): Schmerzfreiheit, Wohlbefinden
b) klinisch: Bei teilweise geringer Größenzunahme, aber auch Größenabnahme der Tumorbereiche, manchmal binnen Stunden, manchmal nach Tagen, teilweise auch völligem Verschwinden von Tumoren und deren Metastasen nach Monaten: Wiederherstellung der groben Kraft, Normalisierung der Sauerstoffaufnahme und -verwertung, sichtbar gesundes Aussehen, Normalisierung der Leberfunktion (Laborwerte), im Bereich des Tumors Hyperthermie bei sonst normaler Körpertemperatur, typisches Schwitzen, Wiederkehren der Nervenfunktion bei Nerven, die durch den Tumor ausgefallen waren, Rückgang der entzündlichen Reaktion im Umfeld der Tumoren, nach unfreiwilligem Placeboversuch (20 Tage), während dessen Dauer es zu einem verstärkten Anwachsen der Metastasengröße in der Lunge und schwerer Atemnot gekommen war, nach erneuter höherdosierter Gabe des Mittels Harnausscheidung von grammweise Tripelphosphat, wonach es wieder zu einer deutlichen Besserung der Atmung und des Allgemeinzustands kam.

Die einzelnen Phasen der Diagnostik und Behandlung lassen sich in einem einzelnen Fall wie folgt beschreiben:

### 1. Phase:

Der Patient, H. A., war 67 Jahre alt. Im Januar 1981 wurde die Diagnose "Karzinom der Retrolingual-Tonsillar-Region" gestellt. Der histologische Befund lautete: Plattenepithelkarzinom, mäßig stark verhornend und mäßig differenziert. Das klinische Stadium war T₃N₂M₀. Einige Tage später folgte die typische Operation mit Tumorresektion, teilweiser Entfernung des Unterkieferknochens und radikaler "neck-dissection" der linken Seite. Die Resektion erreichte nicht überall gesundes Gewebe. Danach erhielt der Patient die typische Bestrahlungsbehandlung mit einer mittleren Dosis von insgesamt 60 Gy. Eine Woche nach dem Ende der Radiotherapie kam es zu einer Blutung aus Oesophagusvarizen, Grad IV, die konservativ beherrscht wurde. Im Februar 1982 wurde eine en-bloc-Nachresektion eines örtlichen Tumorrezidivs mit submentaler Lymphadenektomie durchgeführt. Der Histologiebefund war derselbe. Danach wurde eine örtliche Röntgenbestrahlungsbehandlung mit einer Herddosis von 30 Gy angewendet.

Im März 1982 wurde wegen einer ausgeprägten Schwellung des Zungenrestes oral Adjuvans-Cocktail aus Ethanol 96% : Acetaldehyd puriss. im Verhältnis 1000 ml : 40 ml in einer täglichen Menge von 50 ml in Wasser verabreicht. Begleitend wurde eine glucosearme Diät eingehalten. Das brachte die gewünschte Erleichterung. Die Leukocytenzahl - vorher zwischen 3000 bis 4000/µl - stieg unter dieser Behandlung auf 4000 bis 5000/µl. Es wurden Lymphocytenreizformen (Rieder), lymphatische Plasmazellen und junge Monocyten bis zu 4% im weißen Differentialblutbild gefunden.

### 2. Phase:

Im Juli 1982 wurde ein kirschgroßer Tumorknoten in der linken Submentalregion tastbar. Der Patient inhalierte drei Wochen lang täglich zur Immunstimulation eine Suspension von Liposomen mit MDP. Die Leukocytenzahl stieg auf Zahlen um 5000 pro µl.

Im weißen Differentialblutbild stiegen die Riederzellen und die jungen Monocyten auf 6%. Nach diesen drei Wochen wurde der Tumorknoten entfernt. Der Histologiebefund zeigte neben dem bekannten Karzinom eine signifikante Makrophageneiterung.

### 3. Phase:

Nach 14 Tagen ohne Behandlung wurden im monatlichen Kontrollröntgenbild der Lunge erstmals Tumormetastasen von Wachteleigröße gefunden. Sonographisch wurden Lebermetastasen und eine Milzvergrößerung festgestellt. Hierauf wurde sofort eine cytostatische Chemotherapie über fünf Tage eingeleitet.

Eine zweite Serie, zwei Monate später, mußte nach dem ersten Behandlungstag wegen lebensbedrohlicher Komplikationen abgebrochen werden. Zwischen den Chemotherapieserien und danach wurden diskontinuierlich der oben erwähnte Adjuvans-Cocktail und immunstimulierende Inhalationen verabreicht.

### 4. Phase:

Seit Februar 1983 wurde täglich der Cocktail, die glucosearme Diät und eine Inhalation von Liposomen, die Immunmodulator aus Nocardia enthalten, angewandt. Die Leukocytenzahl stieg auf durchschnittlich 6000/µl. Das weiße Differentialblutbild zeigte Riederlymphocyten und junge Monocyten bei 15%. Diese zwei Zelltypen fielen in ihrer Anzahl relativ, wenn die Leukocytenzahl auf 9000/µl anstieg, und vermehrten sich, wenn die Leukocytenzahl bei 4000/µl lag.

Es wurde eine tägliche Rate von Riederlymphocyten und jungen Monocyten um 900/µl Blut gefunden. Eine pflaumengroße Metastase, die sich in der horizontalen Narbe am linken Hals im Januar 1983 entwickelt hatte, verschwand im April 1983 ohne Ausbildung zusätzlicher Narben. Der Eiter enthielt Makrophagen. Dasselbe wurde bei einer anderen Haut- und einer Lymphknotenmetastase festgestellt. Die Rundschatten in den Lungen-Röntgenbildern wurden kontinuierlich größer von Februar 1983 bis Ende Mai 1983.

Der Patient gab im selben Zeitraum an, schmerzfrei zu sein und sich wohl zu fühlen. Wegen der kontinuierlich produzierten Makrophagenmenge führte das Größerwerden der Lungemetastasen ohne Ausbildung von neuen Metastasen zu dem Gedanken, das Wachstum könnte teilweise auf eine Kapselbildung durch Makrophagen um die Metastasen bedingt sein.

Am 3. Mai 1983 unterzog sich der Patient einem szintigraphischen Test nach Inhalation von mit Immunmodulator aus Nocardia beschickten Liposomen, markiert mit 99 m Tc. Das Aerosol enthielt 1,85 GBq (50 mCi). Der Patient wurde mit einer Nuklear-Chicago-γ-Kamera abgetastet, die mit einem high-resolution-140-KeV-parallel-collimator und einem Simis-3-data-system (Informatek, Birmingham, Alabama, USA) ausgestattet ist. 10-Sekunden-dynamische Abtastungen wurden kontinuierlich eine Stunde lang ausgeführt.

Aus den entstehenden Bildern wurde für den interessierenden Bereich die Lokalisation der Radioaktivität bestimmt. Eine Stunde nach der Anwendung der ^{99m}Tc-Liposomen per Aerosol wurden 1,5% der Radioaktivität in der Lunge gefunden. Der Rest ist in den oberen Atemwegen und im Larynx gefunden worden. Die Computersubtraktion der Hintergrundaktivität erlaubt eine klare Sichtbarmachung der Metastasen. Der Liposomtracer ist vorherrschend in der präbronchialen Region vorhanden. Er zeigt eine Verteilungsasymmetrie, die sehr genau den Röntgenbildern entspricht.

Zu diesem Zeitpunkt fanden sich ungefähr 5% der Lungenradioaktivität im Kreislauf, wie die γ-Kamera-Abtastung des Patienten erwies. Vier Stunden nach der Inhalation der ^{99m}Tc-Liposomen-Suspension wurde eine gesteigerte Radioaktivität im Kreislauf und im Verdauungstrakt gefunden. Das mag bedingt sein durch die Dissoziation des 99 m Tc und die Bindung dieses Tracers an Moleküle, die in der Lage sind, Kapillarwände zu durchqueren. Zu keinem Zeitpunkt fand sich eine Konzentration von Radioaktivität in der Leber. Im Juni 1983 konnten Lebermetastasen sonographisch nicht festgestellt werden.

### 5. Phase:

Im September 1983 kam es zu einem ausgeprägten Schub der schon vor der Krebserkrankung bekannten Leberzirrhose. Nach einer dadurch bedingten 14tägigen Behandlungsunterbrechung zeigte sich sonographisch eine Lebermetastase von etwas unter 3 cm Durchmesser. Im Röntgenkontrollbild der Lunge fanden sich neben luftgefüllten Strukturen in im Abbau befindlichen alten Lungenmetastasen wenige bohnengroße neue Metastasen.

Für eine ausschließliche Behandlung mit beispielsweise Ethanol/Acetaldehyd in wäßriger Lösung und immunmodulatorbeschickten Liposomen ist eine bösartige Tumormasse, zum Beispiel Melanosarkom, von insgesamt 150 g bei guter Immunreaktion des Patienten und einer Laufzeit der Behandlung von einem halben Jahr heilbar, falls der Tumor keine Schutzmechanismen gegen die neue Situation erwirbt.

Durch freies ^{99m}Tc kam es in einem Schilddrüsenadenom zur Anreicherung von Radioaktivität, die eliminierbar war. Nach der Eliminierung des freien 99 m Tc zeigte sich in diesem Adenom der Schilddrüse eine Metastase eines Melanosarkoms.

Die Diagnostik wurde im folgend erstgenannten Fall mit 1,49 GBq-(40 mCi-) und im zweiten angeführten Fall mit 0,93 GBq-(25 mCi-)Markierung durchgeführt.

In einem Fall einer Patientin mit einem Melanosarkom wurden im szintigraphischen Test ein bekannter bohnengroßer Melanomknoten in der Muskulatur des linken Nackens, ein weiterer bekannter Melanomknoten in einem Adenom der rechten Schilddrüse und ein ca. gerstenkorngroßer Melanomknoten im praevertebralen Fettgewebe in Thoraxmitte gefunden. Durch die Behandlung waren in diesem Fall Lokalmetastasen des Melanoms am linken Oberschenkel, Leistenlymphknotenmetastasen beiderseits und paraaortale Lymphknotenmetastasen zurückgegangen.

Bei dieser Patientin bildeten sich szintigraphisch die Gebärmutterinnenseite bei bestehender Regelblutung und die brustwarzennahen Enden der Milchgänge ab, ein pathologisches Geschehen ist dort nicht vorhanden. In einem Fall einer anderen Patientin, bei der einige Wochen vor dem Test ein Melanosarkom des rechten Fußes und ein Lymphknotenpaket mit Metastasen aus dem rechten Oberschenkel histologisch im Gesunden nachreseziert worden war, fanden sich im scintigraphischen Test im rechten und im linken Oberschenkel im gesamten Lymphgebiet kleinste Zellansammlungen des Sarkoms, außerdem Sarkomzellaufschmierungen im Arthrosebereich beider Knie, eine Gefäßdarstellung ergab keinerlei Blutgefäßzusammenhang mit den Tumorzellhäufchen. Die Resorptionsbereiche der Sarkomnachresektionsstellen zeigten trotz noch vorhandenen Ödems keine Strahlung. Ebensowenig bildeten sich ab: eine frische Entzündung von der Venenpunktion vom Vortage, eine resorptive Entzündung um Nahtmaterial in der Tiefe der linken Hohlhand und die beiden arthrotischen Hüftgelenke. Nach zweistündiger Sauerstoffgabe am Abend nach der Szintigraphie hatte sich bis zum Morgen des folgenden Tages eine entzündliche Reaktion im Bereich der strahlenmarkierten Stellen entwickelt im Sinne einer Bestrahlungsüberreaktion.

Bei der auf der vorhergehenden Seite als erste beschriebenen Patientin wurde nach dem Test eine dreimonatige Behandlung mit Cocktail und ImmL täglich durchgeführt, danach wurde erneut ein Test mit SmIL nach Cocktailgabe durchgeführt, der nur noch, deutlich kleiner, den Melanomknoten im Schilddrüsenadenom abbildete. Das Schilddrüsenadenom mit dem Melanomknoten und der Lymphknoten in der Muskulatur des linken Nackens wurden anschließend an den Test chirurgisch entfernt. Die Histologie konnte in dem Knoten in der Schilddrüse nur noch einige wenige Melanomzellen und in dem Knoten im Nacken keine Melanomzellen feststellen. Die Autoradiographie war beim Knoten im Schilddrüsenadenom deutlich positiv, im Knoten aus dem linken Nacken schwach positiv. Die markierten Zellen erscheinen morphologisch zum Teil normal.

Dieselbe Normalisierung der Morphe von Melanomzellen war histologisch am Rande eines Melanoms nach Cocktailgabe vor einer anderen operativen Entfernung beobachtet worden (klinisch und histologisch). Dort war vor der Cocktailgabe der melanotisch gefärbte Bereich markiert worden, 12 Stunden später lag der melanotisch gefärbte Bereich fast einen Zentimeter innerhalb der Markierung.

Diese Patientin ist nun seit fünf Monaten ohne ImmL rezidivfrei. Die Cocktailbehandlung und Diät wurde bei ihr im Finalstadium vor 9 Jahren begonnen. Die Patientin ist seit 8 Jahren wieder berufstätig.

Bei der auf der vorhergehenden Seite als zweiter beschriebenen Patientin kam es fünf Monate nach der Bestrahlungsüberreaktion zu einem Rezidiv in Kirschgröße leistennah am rechten Oberschenkel. Innerhalb eines Monats wuchs der Knoten auf die dreifache Größe an. Er wurde chirurgischerseits teilweise entfernt. Binnen drei Wochen kam es zu einem Lokalrezidiv derselben Größe wie vor der Operation und im Abstand von 5 bis 10 Zentimeter bildeten sich zwei Metastasen in Kirschgröße.

Das Computertomogramm zeigte zusätzlich eine Verdichtung im kleinen Becken rechts. Die Patientin zeigte klinisch die Anzeichen einer Stauung von Gallenwegen in der Leber.

Beim Test mit Cocktail und SmIL zeigte sich eine deutliche Anreicherung scintigraphisch im rechten Oberschenkel und im rechten kleinen Becken.

Durch feinnadelbioptische und saugbioptische histologische und autoradiographische Kontrolle konnte nach Sauerstoffinsufflation über mehrere Stunden schon nach wenigen Tagen als Folge der Anbindung des Strahlers durch den Immunmodulator an die Krebszellwände eine ständig steigende Zahl von geschädigten und nekrotischen Melanomzellen und ein Ansteigen der lokal vorhandenen Lymphozyten, Makrophagen und Granulozyten gefunden werden. Der Effekt geht mit lokaler Hyperthermie, allgemeiner Temperatursteigerung auf 38,5 °C für die Dauer von zwei Tagen und subjektiv mit "leichtem Grippegefühl" einher. Nach viermaliger solcher Therapie in 14 Tagen begannen die klinisch sichtbaren Metastasen in Fibrosierung überzugehen, die intrahepatische Gallenwegsstauung war aufgehoben.

### BEISPIEL 1

### Herstellung eines markierten Immunmodulators:

Zu 30 µg eines im Handel erhältlichen Immunmodulators, der zusammen mit Zinnchlorid in geringem Überschuß zur Anzahl der Aminogruppen des Immunmodulators in lyophilisierter Form unter Stickstoff vorliegt, werden bei Zimmertemperatur 3,7 GBq (100 mCi) ^{99m}Tc in physiologischer Kochsalzlösung zugesetzt. Das Reaktionsgemisch wird 5 Minuten stehen gelassen.

### Unter Stickstoff (N₂):

Getrennt werden 30 µg des Immunmodulators auf 5 mg Liposomen suspendiert in 2,5 ml physiologischer Kochsalzlösung aufgebracht. Die beiden Mischungen werden zusammengegeben, vermischt und 10 Minuten stehen gelassen. Das erhaltene Produkt kann direkt für die Diagnose und Therapie verwendet werden.

### BEISPIEL 2

### Herstellung von Immunmodulator, der mit einem Farbstoff markiert ist:

Herstellung von 1000 Dosen für die Farbdiagnostik malignen Tumorgewebes.
1,9 mg Fluoresceinisothiocyanat (Molekulargewicht 389,4) werden in 50 ml reinem Ethanol gelöst und mit 30 mg Immunmodulator aus Nocardia opaca, gelöst in 5000 ml Wasser, vermischt und bei 37°C eine Stunde stehen gelassen. Um das angefärbte Produkt von nicht umgesetzten Farbstoff zu trennen, gibt man es auf eine Trennsäule von einem Querschnitt von 18 mm, die 50 g Sephadex® G-50 enthält. Mit physiologischer Kochsalzlösung gepuffert auf pH 8 wird eluiert, der nicht umgesetzte Farbstoff wird an der Trennsäule zurückgehalten. Dann wird das Eluat lyophilisiert, es ergibt ca. 1000 Dosen.

Zur Herstellung der Farbidagnostiklösung wird vom Verbraucher aus der Durchstechflasche, die 5 mg einer Lipidmischung entsprechend ≈1 mmol einer Lipidmischung aus Phosphatidylcholin : Phosphatidylserin : Cholesterol im molaren Verhältnis 8 : 2 : 10 in Form von Liposomen mit Radii unter 30 nm (300 Å), suspendiert in 5 ml physiologischer Kochsalzlösung, enthält, der Inhalt mit einer Spritze entnommen und in die Durchstechflasche mit 32 µg des lyophilisierten Fluoresceinisothiocyanat-markierten Immunmodulators aus Nocardia opaca gefüllt. Außer den angegebenen Substanzen befindet sich in den Durchstechflaschen Stickstoff (wie auch in den Durchstechflaschen, die für die Diagnostik und Therapie mit 30 µg Immunmodulator auf 5 mg Liposomen von o.g. Zusammensetzung und Größe, die in 5 ml physiologischer Kochsalzlösung suspendiert sind; wie auch in den Durchstechflaschen, die in lyophilisierter Form Zinnchlorid und den Immunmodulator enthalten). Die erhaltene Lösung läßt man 10 bis 20 Minuten stehen, dann ist sie gebrauchsfertig. Die Mischung und das Stehenlassen findet im schwarzen Versandkarton statt, - UV-Licht setzt die Effizienz des Fluoresceins vorzeitig herab! 1 Mol des Immunmodulators bindet mindestens 10 Mol des Fitc.

### BEISPIEL 3

### Verschiedene Anwendungsformen:

(a) 30 µg Immunmodulator aus Nocardia opaca lyophilisiert, im Handel erhältlich.
(b) Es werden 5 mg Lipidmischung, Phosphatidylcholin : Phosphatidylserin : Cholesterol im molaren Verhältnis von 8 : 2 : 10 hergestellt.
   Diese Lipidmischung liegt vorzugsweise in Form von Liposomen mit Radii unter 30 nm (300 Å), suspendiert in 2,5 bis 5 ml physiologischer Kochsalzlösung vor.
(c) 32 µg lyophilisierter Fluoresceinisothiocyanatmarkierter Immunmodulator aus Nocardia opaca.
(d) 1,1 µg Zinnchlorid (SnCl₂ x 2H₂O) zusammen mit 30 µg Immunmodulator aus Nocardia opaca, lyophilisiert.

Die Proben (a), (b), (c) und (d) können in Durchstechflaschen vorliegen, die die Substanz steril unter Stickstoff (N₂) enthalten.

Die Substanzen (a) und (b) können zur Therapie mit einer Doppelkanüle gemischt werden. Es ist aber auch möglich die Substanzen vorher zu mischen und diese in gemischtem Zustand zu lagern und dann für die Therapie zu verwenden.

Die Proben (a), (b) und (d) werden für den nuklearmedizinischen Test verwendet, indem man zu der Probe (d) 3,7 GBq (100 mCi) 99 mTc in 1 ml physiologischer Kochsalzlösung gibt, dann die Proben (a) und (b) entweder mit der Doppelkanüle mischt oder bereits als Mischung nimmt, und dann wird die Mischung zu der Lösung (d) in der Doppelkanüle zugegeben.

Mit einer solchen Kombination wird auch die lokale Strahlenbehandlung durchgeführt.

Man kann weiterhin die Proben (b) und (c) zum Farbtest mit der Doppelkanüle mischen.

## Patentansprüche (Patentansprüche für folgende(n) Vertragsstaat(en): BE, CH, DE, FR, GB, IT, LI, LU, NL, SE)

1. Mittel zur Diagnose und Therapie von bösartigen Tumoren sowie zur Therapie von Schwächen der zelligen und humoralen Immunabwehr, dadurch **gekennzeichnet,** daß es neben üblichen Trägerstoffen und/oder Verdünnungsmitteln mindestens eine Verbindung aus der Gruppe
(1) einem markierten Immunmodulator als spezifischer Träger für Substanzen durch zellige Gewebe hindurch zu gegebenenfalls vorhandenen Krebszellen zum dortigen Verbleib bei enteraler oder parenteraler Gabe,
(2) einem mit einem radioaktiven Strahler, einem Farbstoff oder einem Cytostatikum markierten Immunmodulator oder einem Gemisch derartig markierter Immunmodulatoren als spezifische Träger für Substanzen durch zellige Gewebe hindurch zu gegebenenfalls vorhandenen Krebszellen zum dortigen Verbleib bei enteraler oder parenteraler Gabe oder
(3) einem mit einem radioaktiven Strahler, einem Farbstoff oder einem Cytostatikum markierten Immunmodulator oder einem Gemisch derartig markierter Immunmodulatoren
(a) in und/oder auf Liposomen als spezifische Träger für Substanzen durch zellige Gewebe hindurch zu gegebenenfalls vorhandenen Krebszellen zum dortigen Verbleib bei enteraler oder parenteraler Gabe oder
(b) lipidiert als spezifische Träger für Substanzen durch zellige Gewebe hindurch zu gegebenenfalls vorhandenen Krebszellen zum dortigen Verbleib bei enteraler oder parenteraler Gabe oder
(4) einem Gemisch aus zwei oder mehreren der obigen Verbindungen als spezifische Träger für Substanzen durch zellige Gewebe hindurch zu gegebenenfalls vorhandenen Krebszellen zum dortigen Verbleib bei enteraler oder parenteraler Gabe
enthält.

2. Mittel nach Anspruch 1, dadurch **gekennzeichnet,** daß es zusammen mit einem Aldehyd der Formel I:
RCHO (I)
worin R ein Wasserstoffatom oder eine geradkettige oder verzweigte Kohlenwasserstoffgruppe mit 1 bis 4 Kohlenstoffatomen bedeutet, sowie gegebenenfalls einem Alkohol der Formel II:
RCH₂OH (II)
worin R die oben angegebene Bedeutung besitzt, verwendet wird.

3. Mittel nach Anspruch 1 oder 2, dadurch **gekennzeichnet,** daß es als Immunmodulator Muramylsäuredipeptidderivate, Peptidoglykane, peptidoglykanfreie Extrakte aus Bakterien, zum Beispiel von Nocardia rubra, Nocardia opaca, Bacillus Calmette-Guérin (BCG) oder Salmonella, oder ihre Gemische enthält.

4. Mittel nach Anspruch 1, 2 oder 3, dadurch **gekennzeichnet,** daß es einen Immunmodulator enthält, der synthetisch oder halbsynthetisch hergestellt worden ist.

5. Mittel nach mindestens einem der vorhergehenden Ansprüche, dadurch **gekennzeichnet,** daß es zusätzlich ein weiteres Agens enthält, das die Sauerstoffabgabe im Bereich maligner Tumore unter Heraufsetzung der therapeutischen Bestrahlungswirkung steigert.

6. Mittel nach Anspruch 5, dadurch **gekennzeichnet,** daß es als Agens zur Steigerung der Sauerstoffabgabe Inositolhexaphosphat, -pentaphosphat, -tetraphosphat oder -triphosphat oder Glycerindiphosphat in einer Zubereitung enthält, die den Einbau dieser Stoffe in Erythrocyten bewirkt.

7. Erzeugnis, enthaltend
a) ein Hilfsmittel, welches neben üblichen pharmazeutisch annehmbaren Trägerstoffen und/oder Verdünnungsmitteln einen Aldehyd der Formel I
RCHO (I)
worin R ein Wasserstoffatom oder eine geradkettige oder verzweigte Kohlenwasserstoffgruppe mit 1 bis 4 Kohlenstoffatomen bedeutet, wobei der freie Aldehyd auch direkt oder indirekt von Stoffen metabolisch freigesetzt werden kann, und gegebenenfalls einen Alkohol der Formel II:
RCH₂OH (II)
worin R die oben angegebene Bedeutung besitzt,
und
b) mindestens eine Verbindung aus der Gruppe
(1) einem Immunmodulator, markiert, als spezifischer Träger für Substanzen durch zellige Gewebe hindurch zu gegebenenfalls vorhandenen Krebszellen zum dortigen Verbleib bei enteraler oder parenteraler Gabe,
(2) einem mit einem radioaktiven Strahler, einem Farbstoff oder einem Cytostatikum markierten Immunmodulator oder einem Gemisch derartig markierter Immunmodulatoren als spezifische Träger für Substanzen durch zellige Gewebe hindurch zu gegebenenfalls vorhandenen Krebszellen zum dortigen Verbleib bei enteraler oder parenteraler Gabe oder
(3) einem mit einem radioaktiven Strahler, einem Farbstoff oder einem Cytostatikum markierten Immunmodulator oder einem Gemisch derartig markierter Immunmodulatoren
(a) in und/oder auf Liposomen als spezifische Träger für Substanzen durch zellige Gewebe hindurch zu gegebenenfalls vorhandenen Krebszellen zum dortigen Verbleib bei enteraler oder parenteraler Gabe oder
(b) lipidiert als spezifische Träger für Substanzen durch zellige Gewebe hindurch zu gegebenenfalls vorhandenen Krebszellen zum dortigen Verbleib bei enteraler oder parenteraler Gabe oder
(4) einem Gemisch aus zwei oder mehreren der obigen Verbindungen als spezifische Träger für Substanzen durch zellige Gewebe hindurch zu gegebenenfalls vorhandenen Krebszellen zum dortigen Verbleib bei enteraler oder parenteraler Gabe
enthält, zur gleichzeitigen, getrennten oder zeitlich abgestuften Anwendung bei der Diagnose und Therapie von malignen Tumoren und zur Therapie von Immunabwehrschwächen.

8. Erzeugnis nach Anspruch 7, dadurch **gekennzeichnet,** daß das Hilfsmittel den Alkohol und den Aldehyd im Verhältnis von 1000 g Alkohol auf 15 bis 40 g Aldehyd enthält.

9. Erzeugnis nach einem der Ansprüche 7 oder 8, dadurch **gekennzeichnet,** daß das Hilfsmittel a) in einer für die orale oder parenterale Verabreichung geeigneten Form und das Mittel (b) in einer für die orale oder parenterale Verabreichung geeigneten Form vorliegen.

10. Erzeugnis nach mindestens einem der Ansprüche 7, 8 oder 9, dadurch **gekennzeichnet,** daß das Hilfsmittel a) in einer für die orale Verabreichung geeigneten Form und das Mittel b) in einer Form, die für die Verabreichung durch Inhalation geeignet ist, vorliegen.

11. Verwendung eines Immunmodulators zur Herstellung eines Mittels als spezifischer Träger für Substanzen durch zellige Gewebe hindurch zu gegebenenfalls vorhandenen Krebszellen zum dortigen Verbleib bei enteraler oder parenteraler Gabe.

12. Verwendung nach Anspruch 11, dadurch **gekennzeichnet,** daß der Immunmodulator zur Herstellung eines Mittels als Träger für einen radioaktiven Strahler, einen Farbstoff oder ein Zytostatikum verwendet wird.

13. Verwendung nach Anspruch 11 oder 12, dadurch **gekennzeichnet**, daß der Immunmodulator in und/oder auf Liposomen oder lipidiert verwendet wird.

14. Verwendung eines Immunmodulators zur Herstellung eines Mittels zur Diagnose und Therapie von bösartigen Tumoren sowie zur Therapie von Schwächen der zelligen und humoralen Immunabwehr als spezifischer Träger für Substanzen durch zellige Gewebe hindurch zu gegebenenfalls vorhandenen Krebszellen zum dortigen Verbleib bei enteraler oder parenteraler Gabe.

## Patentansprüche (Patentansprüche für folgende(n) Vertragsstaat(en): AT)

1. Verfahren zur Herstellung eines Mittels zur Diagnose und Therapie von bösartigen Tumoren sowie zur Therapie von Schwächen der zelligen und humoralen Immunabwehr, dadurch **gekennzeichnet,** daß man mit üblichen Trägerstoffen und/oder Verdünnungsmitteln mindestens eine Verbindung aus der Gruppe
(1) einem markierten Immunmodulator als spezifischer Träger für Substanzen durch zellige Gewebe hindurch zu gegebenenfalls vorhandenen Krebszellen zum dortigen Verbleib bei enteraler oder parenteraler Gabe,
(2) einem mit einem radioaktiven Strahler, einem Farbstoff oder einem Cytostatikum markierten Immunmodulator oder einem Gemisch derartig markierter Immunmodulatoren als spezifische Träger für Substanzen durch zellige Gewebe hindurch zu gegebenenfalls vorhandenen Krebszellen zum dortigen Verbleib bei enteraler oder parenteraler Gabe oder
(3) einem mit einem radioaktiven Strahler, einem Farbstoff oder einem Cytostatikum markierten Immunmodulator oder einem Gemisch derartig markierter Immunmodulatoren
(a) in und/oder auf Liposomen als spezifische Träger für Substanzen durch zellige Gewebe hindurch zu gegebenenfalls vorhandenen Krebszellen zum dortigen Verbleib bei enteraler oder parenteraler Gabe oder
(b) lipidiert als spezifische Träger für Substanzen durch zellige Gewebe hindurch zu gegebenenfalls vorhandenen Krebszellen zum dortigen Verbleib bei enteraler oder parenteraler Gabe oder
(4) einem Gemisch aus zwei oder mehreren der obigen Verbindungen als spezifische Träger für Substanzen durch zellige Gewebe hindurch zu gegebenenfalls vorhandenen Krebszellen zum dortigen Verbleib bei enteraler oder parenteraler Gabe
vermischt.

2. Verfahren nach Anspruch 1, dadurch **gekennzeichnet,** daß das Gemisch mit einem Aldehyd der Formel I:
RCHO (I)
worin R ein Wasserstoffatom oder eine geradkettige oder verzweigte Kohlenwasserstoffgruppe mit 1 bis 4 Kohlenstoffatomen bedeutet, sowie gegebenenfalls einem Alkohol der Formel II:
RCH₂OH (II)
worin R die oben angegebene Bedeutung besitzt, zusammengebracht wird.

3. Verfahren nach Anspruch 1 oder 2, dadurch **gekennzeichnet,** daß man als Immunmodulator Muramylsäuredipeptidderivate, Peptidoglykane, peptidoglykanfreie Extrakte aus Bakterien, zum Beispiel von Nocardia rubra, Nocardia opaca, Bacillus Calmette-Guérin (BCG) oder Salmonella, oder ihre Gemische verwendet.

4. Verfahren nach Anspruch 1, 2 oder 3, dadurch **gekennzeichnet,** daß man einen Immunmodulator verwendet, der synthetisch oder halbsynthetisch hergestellt worden ist.

5. Verfahren nach mindestens einem der vorhergehenden Ansprüche, dadurch **gekennzeichnet,** daß man ein weiteres Agens verwendet, das die Sauerstoffabgabe im Bereich maligner Tumore unter Heraufsetzung der therapeutischen Bestrahlungswirkung steigert.

6. Verfahren nach Anspruch 5, dadurch **gekennzeichnet,** daß man als Agens zur Steigerung der Sauerstoffabgabe Inositolhexaphosphat, -pentaphosphat, -tetraphosphat oder -triphosphat oder Glycerindiphosphat in einer Zubereitung verwendet, die den Einbau dieser Stoffe in Erythrocyten bewirkt.

7. Verwendung eines Immunmodulators zur Herstellung eines Mittels zur Diagnose und Therapie von bösartigen Tumoren sowie zur Therapie von Schwächen der zelligen und humoralen Immunabwehr als spezifischer Träger für Substanzen durch zellige Gewebe hindurch zu gegebenenfalls vorhandenen Krebszellen zum dortigen Verbleib bei enteraler oder parenteraler Gabe.

## Claims (Claims for the following Contracting State(s): BE, CH, DE, FR, GB, IT, LI LU, NL, SE)

1. An agent for the diagnosis and therapy of malignant tumours and for the therapy of weaknesses of the cellular and humoral immune defence, characterised in that it contains, in addition to conventional carriers and/or diluents, at least one compound from among
(1) a labelled immunomodulator as specific carrier for substances through cellular tissue to cancerous cells possibly present to remain there, administration being enteral or parenteral,
(2) a labelled immunomodulator labelled with a radioactive emitter, with a stain or with a cytostatic agent, or a mixture of such labelled immunomodulators, as specific carriers for substances through cellular tissue to cancerous cells possibly present to remain there, administration being enteral or parenteral, or
(3) a labelled immunomodulator labelled with a radioactive emitter, with a stain or with a cytostatic agent, or a mixture of such labelled immunomodulators
(a) in and/or on liposomes as specific carriers for substances through cellular tissue to cancerous cells possibly present to remain there, administration being enteral or parenteral, or
(b) lipidised as specific carriers for substances through cellular tissue to cancerous cells possibly present to remain there, administration being enteral or parenteral, or
(4) a mixture of two or more of the above compounds as specific carriers for substances through cellular tissue to cancerous cells possibly present to remain there, administration being enteral or parenteral.

2. An agent according to claim 1, characterised in that it is used together with an aldehyde corresponding to formula I:
RCHO (I)
wherein R denotes a hydrogen atom or a straight-chain or branched hydrocarbon group having 1 to 4 carbon atoms, and optionally together with an alcohol corresponding to formula II:
RCH₂OH (II)
wherein R has the meaning given above.

3. An agent according to claim 1 or 2, characterised in that it contains as immunomodulators muramyl dipeptide derivatives, peptidoglycans, peptidoglycan-free extracts of bacteria, for example, of Nocardia rubra, Nocardia opaca, Bacillus Calmette-Guérin (BCG) or Salmonella, or mixtures thereof.

4. An agent according to claim 1, 2 or 3, characterised in that it contains an immunomodulator which has been prepared synthetically or semi-synthetically.

5. An agent according to at least one of the preceding claims, characterised in that it contains in addition another agent, which increases the release of oxygen in the region of malignant tumours, accompanied by an increase in the therapeutic radiative action.

6. An agent according to claim 5, characterised in that the agent that it contains for increasing the release of oxygen is inositol hexaphosphate, inositol pentaphosphate, inositol tetraphosphate or inositol triphosphate or glycerol diphosphate in a formulation which brings about the incorporation of these substances into erythrocytes.

7. A product, containing
a) an auxiliary substance which contains, in addition to conventional pharmaceutically tolerable carriers and/or diluents, an aldehyde corresponding to formula I:
RCHO (I)
wherein R denotes a hydrogen atom or a straight-chain or branched hydrocarbon group having 1 to 4 carbon atoms, the free aldehyde also being directly or indirectly metabolically releasable from materials, and optionally an alcohol corresponding to formula II:
RCH₂OH (II)
wherein R has the meaning given above, and
b) at least one compound from among
(1) a labelled immunomodulator as specific carrier for substances through cellular tissue to cancerous cells possibly present to remain there, administration being enteral or parenteral,
(2) a labelled immunomodulator labelled with a radioactive emitter, with a stain or with a cytostatic agent, or a mixture of such labelled immunomodulators, as specific carriers for substances through cellular tissue to cancerous cells possibly present to remain there, administration being enteral or parenteral, or
(3) a labelled immunomodulator labelled with a radioactive emitter, with a stain or with a cytostatic agent, or a mixture of such labelled immunomodulators
(a) in and/or on liposomes as specific carriers for substances through cellular tissue to cancerous cells possibly present to remain there, administration being enteral or parenteral, or
(b) lipidised as specific carriers for substances through cellular tissue to cancerous cells possibly present to remain there, administration being enteral or parenteral, or
(4) a mixture of two or more of the above compounds as specific carriers for substances through cellular tissue to cancerous cells possibly present to remain there, administration being enteral or parenteral,
for application simultaneously, separately or at intervals in the diagnosis and therapy of malignant tumours and for the therapy of weaknesses of the immune defence.

8. A product according to claim 7, characterised in that the auxiliary substance contains the alcohol and the aldehyde in the proportion of 1000 g of alcohol to 15 to 40 g of aldehyde.

9. A product according to either of claims 7 or 8, characterised in that the auxiliary substance a) is in a form suitable for oral or parenteral administration and the agent b) is in a form suitable for oral or parenteral administration.

10. A product according to at least one of claims 7, 8 or 9, characterised in that the auxiliary substance a) is in a form suitable for oral administration and the agent b) is in a form suitable for administration by inhalation.

11. The use of an immunomodulator in the preparation of an agent as specific carrier for substances through cellular tissue to cancerous cells possibly present to remain there, administration being enteral or parenteral.

12. The use according to claim 11, characterised in that the immunomodulator is used for the preparation of an agent as carrier for a radioactive emitter, for a stain or for a cytostatic agent.

13. The use according to claim 11 or 12, characterised in that the immunomodulator is used in and/or on liposomes or is lipidised.

14. The use of an immunomodulator in the preparation of an agent for the diagnosis and therapy of malignant tumours and for the therapy of weaknesses of the cellular and humoral immune defence as a specific carrier for substances through cellular tissue to cancerous cells possibly present to remain there, administration being enteral or parenteral.

## Claims (Claims for the following Contracting State(s): AT)

1. A method for the preparation of an agent for the diagnosis and therapy of malignant tumours and for the therapy of weaknesses of the cellular and humoral immune defence, characterised in that at least one compound from among
(1) a labelled immunomodulator as specific carrier for substances through cellular tissue to cancerous cells possibly present to remain there, administration being enteral or parenteral,
(2) a labelled immunomodulator labelled with a radioactive emitter, with a stain or with a cytostatic agent, or a mixture of such labelled immunomodulators, as specific carriers for substances through cellular tissue to cancerous cells possibly present to remain there, administration being enteral or parenteral, or
(3) a labelled immunomodulator labelled with a radioactive emitter, with a stain or with a cytostatic agent, or a mixture of such labelled immunomodulators
(a) in and/or on liposomes as specific carriers for substances through cellular tissue to cancerous cells possibly present to remain there, administration being enteral or parenteral, or
(b) lipidised as specific carriers for substances through cellular tissue to cancerous cells possibly present to remain there, administration being enteral or parenteral, or
(4) a mixture of two or more of the above compounds as specific carriers for substances through cellular tissue to cancerous cells possibly present to remain there, administration being enteral or parenteral
is mixed with conventional carriers and/or diluents.

2. A method according to claim 1, characterised in that the mixture is brought into contact with an aldehyde corresponding to formula I:
RCHO (I)
wherein R denotes a hydrogen atom or a straight-chain or branched hydrocarbon group having 1 to 4 carbon atoms, and optionally with an alcohol corresponding to formula II:
RCH₂OH (II)
wherein R has the meaning given above.

3. A method according to claim 1 or 2, characterised in that muramyl dipeptide derivatives, peptidoglycans, peptidoglycan-free extracts of bacteria, for example, of Nocardia rubra, Nocardia opaca, Bacillus Calmette-Guérin (BCG) or Salmonella, or mixtures thereof are used as immunomodulator.

4. A method according to claim 1, 2 or 3, characterised in that an immunomodulator which has been prepared synthetically or semi-synthetically is used.

5. A method according to at least one of the preceding claims, characterised in that another agent is used, which agent increases the release of oxygen in the region of malignant tumours, accompanied by an increase in the therapeutic radiative action.

6. A method according to claim 5, characterised in that the agent used for increasing the release of oxygen is inositol hexaphosphate, inositol pentaphosphate, inositol tetraphosphate or inositol triphosphate or glycerol diphosphate in a formulation which brings about the incorporation of these substances into erythrocytes.

7. The use of an immunomodulator in the preparation of an agent for the diagnosis and therapy of malignant tumours and for the therapy of weaknesses of the cellular and humoral immune defence as a specific carrier for substances through cellular tissue to cancerous cells possibly present to remain there, administration being enteral or parenteral.

## Revendications (Revendications pour l'(les) Etat(s) contractant(s) suivant(s): BE, CH, DE, FR, GB, IT, LI, LU, NL, SE)

1. Agent pour le diagnostic et la thérapie de tumeurs malignes, ainsi que pour la thérapie des déficiences du système immunitaire cellulaire et humoral, caractérisé en ce qu'il contient, en plus des véhicules et/ou des diluants usuels, au moins un composé choisi dans l'ensemble constitué par :
(1) un immunomodulateur marqué comme support spécifique pour des substances à travers les tissus cellulaires et en direction des cellules cancéreuses éventuellement présentes, pour y séjourner, par administration par voie entérale ou parentérale,
(2) un immunomodulateur marqué au moyen d'un émetteur radioactif, d'un colorant ou d'un agent cytostatique, ou un mélange d'immunomodulateurs marqués de ce type, comme supports spécifiques pour des substances à travers les tissus cellulaires et en direction des cellules cancéreuses éventuellement présentes, pour y séjourner, par administration par voie entérale ou parentérale, ou
(3) un immunomodulateur marqué au moyen d'un émetteur radioactif, d'un colorant ou d'un agent cytostatique, ou un mélange d'immunomodulateurs marqués de ce type,
(a) dans et/ou sur des liposomes comme supports spécifiques pour des substances à travers les tissus cellulaires et en direction des cellules cancéreuses éventuellement présentes, pour y séjourner, par administration par voie entérale ou parentérale, ou
(b) sous une forme lipidique, comme supports spécifiques pour des substances à travers les tissus cellulaires et en direction des cellules cancéreuses éventuellement présentes, pour y séjourner, par administration par voie entérale ou parentérale, ou
(4) un mélange d'au moins deux composés indiqués ci-dessus, comme supports spécifiques pour des substances à travers les tissus cellulaires et en direction des cellules cancéreuses éventuellement présentes, pour y séjourner, par administration par voie entérale ou parentérale.

2. Agent selon la revendication 1, caractérisé en ce qu'on l'utilise conjointement avec un aldéhyde répondant à la formule I :
RCHO (I)
où R signifie un atome d'hydrogène ou un groupe hydrocarbure linéaire ou ramifié ayant de 1 à 4 atomes de carbone, ainsi qu'éventuellement un alcool répondant à la formule II :
RCH₂OH (II)
où R a la signification indiquée ci-dessus.

3. Agent selon la revendication 1 ou 2, caractérisé en ce qu'il contient, comme immunomodulateur, des dérivés dipeptidiques de l'acide muramylique, des peptidoglycanes, des extraits de bactéries dépourvus de peptidoglycanes, par exemple de Nocardia rubra, de Nocardia opaca, le bacille de Calmette-Guérin (BCG) ou des salmonelles, ou des mélanges de ceux-ci.

4. Agent selon la revendication 1, 2 ou 3, caractérisé en ce qu'il contient un immunomodulateur qui est un produit synthétique ou semi-synthétique.

5. Agent selon au moins l'une des revendications précédentes, caractérisé en ce qu'il contient, en outre, un autre agent qui augmente la libération d'oxygène dans la région des tumeurs malignes, tout en augmentant l'effet thérapeutique de l'irradiation.

6. Agent selon la revendication 5, caractérisé en ce qu'il contient, comme agent servant à augmenter la libération d'oxygène, l'hexaphosphate, le pentaphosphate, le tétraphosphate ou le triphosphate d'inositol, ou le diphosphate de glycérol, dans une préparation qui provoque l'incorporation de ces substances dans les érythrocytes.

7. Produit contenant :
a) un adjuvant qui contient, en plus des véhicules et/ou des diluants pharmaceutiques usuels, un aldéhyde répondant à la formule I :
RCHO (I)
où R signifie un atome d'hydrogène ou un groupe hydrocarbure linéaire ou ramifié ayant de 1 à 4 atomes de carbone, l'aldéhyde libre pouvant aussi être libéré de substances directement ou indirectement par voie métabolique, et éventuellement un alcool répondant à la formule II :
RCH₂OH (II)
où R a la signification indiquée ci-dessus, et
b) au moins un composé choisi dans l'ensemble constitué par :
(1) un immunomodulateur marqué comme support spécifique pour des substances à travers les tissus cellulaires et en direction des cellules cancéreuses éventuellement présentes, pour y séjourner, par administration par voie entérale ou parentérale,
(2) un immunomodulateur marqué au moyen d'un émetteur radioactif, d'un colorant ou d un agent cytostatique, ou un mélange d'immunomodulateurs marqués de ce type, comme supports spécifiques pour des substances à travers les tissus cellulaires et en direction des cellules cancéreuses éventuellement présentes, pour y séjourner, par administration par voie entérale ou parentérale, ou
(3) un immunomodulateur marqué au moyen d'un émetteur radioactif, d'un colorant ou d'un agent cytostatique, ou un mélange d'immunomodulateurs marqués de ce type,
(a) dans et/ou sur des liposomes comme supports spécifiques pour des substances à travers les tissus cellulaires et en direction des cellules cancéreuses éventuellement présentes, pour y séjourner, par administration par voie entérale ou parentérale, ou
(b) sous une forme lipidique, comme supports spécifiques pour des substances à travers les tissus cellulaires et en direction des cellules cancéreuses éventuellement présentes, pour y séjourner, par administration par voie entérale ou parentérale, ou
(4) un mélange d'au moins deux composés indiqués ci-dessus, comme supports spécifiques pour des substances à travers les tissus cellulaires et en direction des cellules cancéreuses éventuellement présentes, pour y séjourner, par administration par voie entérale ou parentérale.
destiné à être utilisé simultanément, séparément ou de façon échelonnée dans le temps, pour le diagnostic et la thérapie des tumeurs malignes et pour la thérapie des déficiences du système immunitaire.

8. Produit selon la revendication 7, caractérisé en ce que l'adjuvant contient l'alcool et l'aldéhyde à un rapport de 1 000 g d'alcool pour 15 à 40 g d'aldéhyde.

9. Produit selon la revendication 7 ou 8, caractérisé en ce que l'adjuvant (a) est sous une forme appropriée à l'administration orale ou parentérale, et l'agent (b) est sous une forme appropriée à l'administration orale ou parentérale.

10. Produit selon au moins l'une des revendications 7, 8 ou 9, caractérisé en ce que l'adjuvant (a) est sous une forme appropriée à l'administration orale, et l'agent (b) est sous une forme appropriée à l'administration par inhalation.

11. Utilisation d'un immunomodulateur pour la préparation d'un agent comme support spécifique pour des substances à travers les tissus cellulaires et en direction des cellules cancéreuses éventuellement présentes, pour y séjourner, par administration par voie entérale ou parentérale.

12. Utilisation selon la revendication 11, caractérisée en ce qu'on utilise l'immunomodulateur pour la préparation d'un agent comme support pour un émetteur radioactif, un colorant ou un agent cytostatique.

13. Utilisation selon la revendication 11 ou 12, caractérisée en ce qu'on utilise l'immunomodulateur dans et/ou sur des liposomes ou sous une forme lipidique.

14. Utilisation d'un immunomodulateur pour la préparation d'un agent pour le diagnostic et la thérapie de tumeurs malignes, ainsi que pour la thérapie de déficiences du système immunitaire cellulaire ou humoral, comme support spécifique pour des substances à travers les tissus cellulaires et en direction des cellules cancéreuses éventuellement présentes, pour y séjourner, par administration par voie entérale ou parentérale.

## Revendications (Revendications pour l'(les) Etat(s) contractant(s) suivant(s): AT)

1. Procédé de préparation d un agent pour le diagnostic et la thérapie de tumeurs malignes, ainsi que pour la thérapie des déficiences du système immunitaire cellulaire et humoral, caractérisé en ce qu'on mélange, avec des véhicules et/ou des diluants usuels, au moins un composé choisi dans l'ensemble constitué par :
(1) un immunomodulateur marqué comme support spécifique pour des substances à travers les tissus cellulaires et en direction des cellules cancéreuses éventuellement présentes, pour y séjourner, par administration par voie entérale ou parentérale,
(2) un immunomodulateur marqué au moyen d'un émetteur radioactif, d'un colorant ou d'un agent cytostatique, ou un mélange d'immunomodulateurs marqués de ce type, comme supports spécifiques pour des substances à travers les tissus cellulaires et en direction des cellules cancéreuses éventuellement présentes, pour y séjourner, par administration par voie entérale ou parentérale, ou
(3) un immunomodulateur marqué au moyen d'un émetteur radioactif, d'un colorant ou d'un agent cytostatique, ou un mélange d'immunomodulateurs marqués de ce type,
(a) dans et/ou sur des liposomes comme supports spécifiques pour des substances à travers les tissus cellulaires et en direction des cellules cancéreuses éventuellement présentes, pour y séjourner, par administration par voie entérale ou parentérale, ou
(b) sous une forme lipidique, comme supports spécifiques pour des substances à travers les tissus cellulaires et en direction des cellules cancéreuses éventuellement présentes, pour y séjourner, par administration par voie entérale ou parentérale, ou
(4) un mélange d'au moins deux composés indiqués ci-dessus, comme supports spécifiques pour des substances à travers les tissus cellulaires et en direction des cellules cancéreuses éventuellement présentes, pour y séjourner, par administration par voie entérale ou parentérale.

2. Procédé selon la revendication 1, caractérisé en ce qu'on combine le mélange avec un aldéhyde répondant à la formule I :
RCHO (I)
où R signifie un atome d'hydrogène ou un groupe hydrocarbure linéaire ou ramifié ayant de 1 à 4 atomes de carbone, ainsi qu'éventuellement un alcool répondant à la formule II :
RCH₂OH (II)
où R a la signification indiquée ci-dessus.

3. Procédé selon la revendication 1 ou 2, caractérisé en ce qu'on utilise, comme immunomodulateur, des dérivés dipeptidiques de l'acide muramylique, des peptidoglycanes, des extraits de bactéries dépourvus de peptidoglycanes, par exemple de Nocardia rubra, de Nocardia opaca, le bacille de Calmette-Guérin (BCG) ou des salmonelles, ou des mélanges de ceux-ci.

4. Procédé selon la revendication 1, 2 ou 3, caractérisé en ce qu'on utilise un immunomodulateur qu'on a préparé par synthèse ou semi-synthèse.

5. Procédé selon au moins l'une des revendications précédentes, caractérisé en ce qu'on utilise un agent supplémentaire qui augmente la libération d'oxygène dans la région des tumeurs malignes, tout en augmentant l'effet thérapeutique de l'irradiation.

6. Procédé selon la revendication 5, caractérisé en ce qu'on utilise, comme agent servant à augmenter la libération d'oxygène, l'hexaphosphate, le pentaphosphate, le tétraphosphate ou le triphosphate d'inositol, ou le diphosphate de glycérol, dans une préparation qui provoque l'incorporation de ces substances dans les érythrocytes.

7. Utilisation d'un immunomodulateur pour la préparation d'un agent pour le diagnostic et la thérapie de tumeurs malignes, ainsi que pour la thérapie de déficiences du système immunitaire cellulaire et humoral, comme support spécifique pour des substances à travers les tissus cellulaires et en direction des cellules cancéreuses éventuellement présentes, pour y séjourner, par administration par voie entérale ou parentérale.
